# EUROPÄISCHE PATENTANMELDUNG

(11) **EP 0 986 018 A2**
(43) Veröffentlichungstag der Anmeldung: **15.03.2000**
(21) Anmeldenummer: 99117119.0
(22) Anmeldetag: 31.08.1999
(51) Int. Cl.: G06F 19/00

(54) **System zur Kommunikation zwischen einem Arzt und einem Patienten sowie Verfahren zum Aufnehmen von Anamnesedaten**

(30) Priorität: 09.09.1998 DE 19841221
(71) Anmelder: SIEMENS AKTIENGESELLSCHAFT, 80333 München (DE)
(72) Erfinder: Abraham-Fuchs, Klaus, 91058 Erlangen (DE); Birkhölzer, Thomas, Dr., 91085 Weisendorf (DE); Herold, Alexander, Dr., 91052 Erlangen (DE)

(57) **Zusammenfassung**

System zur Kommunikation zwischen einem Arzt und einem Patienten über eine Kommunikationsleitung, umfassend eine erste arztseitig vorgesehene Datenverarbeitungseinrichtung (1) mit zugeordneten Ausgabemedium (2) und eine zweite patientenseitig vorgesehene Datenverarbeitungseinrichtung (6) mit zugeordneten Ausgabemedium (7), die kanalgebunden miteinander kommunizieren und denen ein Expertensystem (4) zugeordnet ist, das zur interaktiven kanalgebundenen Abfrage und Aufnahme relevanter, insbesondere medizinisch relevanter und vom Patienten entsprechend der vom Expertensystem (4) formulierten, an die zweite Datenverarbeitungseinrichtung (6) gegebenen Anfragen einzugebener Anamnesedaten des Patienten ausgebildet ist, welche bei Bedarf dem Arzt über dessen Ausgabemedium ausgebbar sind.

## Beschreibung

Die Erfindung betrifft ein System zur Kommunikation zwischen einem Arzt und einem Patienten über eine Kommunikationsleitung.

Hat ein Patient ein Problem oder eine Frage, die er von einem Arzt beantwortet haben möchte, so begibt er sich entweder in die ärztliche Sprechstunde, er konsultiert den Arzt also persönlich, ansonsten besteht die Möglichkeit einer telefonischen Beratung. In vielen Fällen jedoch ist medizinischer Rat nicht unmittelbar notwendig, sondern innerhalb einer Zeitspanne von z.B. 24 Stunden ausreichend. Bei solchen subakuten medizinischen Problemen ist es für beide Seiten, also den Patienten und den betreuenden Arzt, nicht zwingend erforderlich, direkt miteinander zu kommunizieren. Der unmittelbare Gesprächskontakt wird jedoch insbesondere von ärztlicher Seite bevorzugt, da hierbei die aus ärztlicher Sicht relevanten Anamnesedaten weitgehend vollständig aufgenommen werden können. Dem gegenüber ist jedoch der persönliche Kontakt mit viel Aufwand verbunden, so muß der Patient beispielsweise seinen Arbeitsplatz verlassen, Wege- und Wartezeit fällt an, zum anderen kann sich der Arzt in dieser Zeit nicht den akuten Problemen anderer Patienten widmen. Auch der mit dem Besuch des Patienten in der Arztpraxis verbundene Verwaltungsaufwand (z.B. Heraussuchen der Patientenakte etc.) fällt an.

Der Erfindung liegt das Problem zugrunde, eine Möglichkeit zur Kommunikation Arzt - Patient anzugeben, die sowohl für den Arzt als auch für den Patienten mit weniger Aufwand verbunden ist.

Zur Lösung dieses Problems ist ein System zur Kommunikation zwischen einem Arzt und einem Patienten über eine Kommunikationsleitung vorgesehen, umfassend eine erste arztseitig vorgesehene Datenverarbeitungseinrichtung mit zugeordnetem Ausgabemedium, und eine zweite patientenseitig vorgesehene Datenverarbeitungseinrichtung mit zugeordnetem Ausgabemedium, die kanalgebunden miteinander kommunizieren und denen ein Expertensystem zugeordnet ist, das zur interaktiven kanalgebundenen Abtrage und Aufnahme relevanter, insbesondere medizinisch relevanter und vom Patienten entsprechend der vom Expertensystem formulierten, an die zweite Datenverarbeitungseinrichtung gegebenen Anfragen einzugebender Anamnesedaten des Patienten ausgebildet ist, welche bei Bedarf dem Arzt über dessen Ausgabemedium ausgebbar sind.

Die Erfindung gibt ein System zur interaktiven Aufnahme von Anamnesedaten an, die der Arzt bei passender Gelegenheit abruft und auf deren Grundlage er seinen Ratschlag formuliert, welchen er dann ebenfalls über die Kommunikationsleitung an den Patienten geben kann, wobei natürlich auch ein direkter telefonischer Antwortkontakt erfolgen kann. Der Patient wählt sich vorteilhaft über seine Datenverarbeitungseinrichtung z.B. bei der arztseitig vorgesehenen Datenverarbeitungseinrichtung ein. Das dort vorgesehene Expertensystem, bei dem es sich um ein intelligentes, wissensbasiertes System handelt, ist im Stande, gezielt Anamnesedaten abzufragen, d.h., das Expertensystem formuliert gezielt Fragen, die über die Kommunikationsleitung, beispielsweise über das Internet oder per e-mail, an den Patienten gegeben werden, der diese dann beantwortet. Es handelt sich also um ein interaktives Frage-Antwort-System, wobei das mit einem sehr breiten Fachwissen belegte Expertensystem die jeweils relevaten Fragen formulieren kann. Nach Sammeln sämtlicher relevanter Anamnesedaten werden diese seitens der ersten Datenverarbeitungseinrichtung gespeichert. Als Anamnesedaten werden alle möglicherweise gesundheitsrelevanten Informationen verstanden, die dem Patienten bekannt sind bzw. bekannt sein könnten (z.B. Vorgeschichte, aktuelle Symptomatik, auch Messwerte wie z.B. Blutdruck oder alte Laborwerte, aber z.B. nicht aktuelle Laborwerte) und die deswegen vom Patienten erfragt werden können. Der Arzt kann nun bei Bedarf, beispielsweise nach Praxisschluß, die eingegangenen Anfragen abrufen und auswerten. Damit ist weder der Patient noch der Arzt primär gezwungen, persönlichen Kontakt aufzunehmen. Handelt es sich um ein subakutes medizinisches Problem, beispielsweise nur die vergessene Einnahme eines Medikamentes oder dergleichen, ist eine Beratung innerhalb einer bestimmten Zeitspanne von z.B. 24 Stunden ausreichend, was beim erfindungsgemäßen System ohne weiteres möglich ist, als der Arzt die Antwort beispielsweise am jeweiligen Abend geben kann. Handelt es sich um ein akuteres Problem, das eine persönliche Konsultation erfordert, kann dies der Arzt aufgrund der vom Expertensystem aufgenommenen, tatsächlich relevanten Anamnesedaten ebenfalls feststellen und es dem Patienten mitteilen. Das erfindungsgemäße System verringert also in beachtlicher Weise den allein mit der Fragestellung und Antwortbildung anfallenden Aufwand beider Seiten, da dies allein durch die interaktive, durch das Expertensystem medizinisch gestützte Kommunikation über die Kommunikationsleitung erfolgt. Das Expertensystem kann aber auch patientenseitig oder in einer dritten, externen Datenverarbeitungseinrichtung, z.B. einem zentralen systemeigenen Server vorgesehen sein, über welchen die Kommunikation läuft. Die Kommunikation kann über beliebige Wege erfolgen, also sowohl leitungsgebunden wie auch drahtlos.

Das Expertensystem kann erfindungsgemäß zur Formulierung einer jeweiligen Anfrage in Abhängigkeit einer oder mehrerer vorher patientenseitig gegebener Daten und Anfragen ausgebildet sein. D.h., das intelligente Expertensystem erkennt den konkreten Aussagegehalt der vom Patienten gegebenen Information und formuliert seine folgende Frage entsprechend der gegebenen Antwort(en), d.h., das System verknüpft die ihm gegebene Information mit vorliegendem Expertenwissen und zieht entsprechende Folgerungen zur Formulierung nachfolgender Fragen. Hierdurch ist es möglich, daß das Expertensystem zielgerichtet Fragen formulieren kann, um auf diese Weise die für den Arzt tatsächlich relevanten Anamnesedaten abzufragen. Im Rahmen dieser gezielten Fragestellungen erhält der Patient ferner sofort ein klares Bild über seine Situation, wobei das Eypertensystem im Einzelfall auch Aussagen über bestimmte vorzunehmende Handlungsalternativen geben kann, was jedoch abhängig vom patientenspezifischen Problem ist.

Ein weiteres Problem, das der Arzt in der Regel durch das persönliche Gespräch ausräumen möchte und kann, ist, daß der Patient das medizinische Problem zumeist medizinisch inkorrekt, mehrdeutig und laienhaft formuliert, d.h., der Arzt muß häufig mehrfach hinterfragen. Um im Rahmen der interaktiven Datenkommunikation auch hier Abhilfe zu schaffen, kann erfindungsgemäß das Expertensystem zum Erkennen und/oder Formulieren und Ausgeben medizinisch eindeutiger Anamnesedaten auf Basis der vom Patienten eingegebenen Daten ausgebildet sein. Das Expertensystem ist also in der Lage, die vom Patienten gegebenen, häufig inkorrekten und laienhaften Informationen in medizinisch eindeutige Aussagen zu "übersetzen", so daß dem Arzt zweifelsfreie Anamnesedaten geliefert werden. Da das Expertensystem wie beschrieben imstande ist, gezielt die an den Patienten gestellten Fragen zu formulieren, ist es möglich, so lange zu hinterfragen, bis ein medizinisch eindeutiger Aussagegehalt erkannt, formuliert und ausgegeben werden kann. Daneben ist es bedingt durch die gezielte Fragemöglichkeit auch möglich, vom Patienten wesentliche Zusatzinformationen mit aufzunehmen, um auf diese Weise ein vollständiges Datenprofil aufzunehmen.

Die Datenkommunikation bietet darüber hinaus noch eine Reihe weiterer vorteilhafter Möglichkeiten. So kann erfindungsgemäß wenigstens einer der Datenverarbeitungseinrichtungen ein Speicherbereich enthaltend patientenspezifische Daten zugeordnet sein. Alternativ, ggf. auch zusätzlich kann der Speicherbereich in einer vierten, externen Datenverarbeitungseinrichtung, die entsprechend in den Kommunikationsbetrieb einbindbar ist, vorgesehen sein. Bei diesen Daten handelt es sich um persönliche Patientendaten, die für die Erstellung des ärztlichen Rates erforderlich sein können. So kann erfindungsgemäß der Speicherbereich patientenseitig vorgesehen sein, wobei die Daten zumindest teilweise automatisch von der ersten Datenverarbeitungseinrichtung bzw. dem Expertensystem abfragbar sind, alternativ oder zusätzlich können die Daten zumindest teilweise bei Bedarf vom Patienten auch erst freigebbar sein, d.h., der Patient muß entscheiden, inwieweit die Daten oder entsprechende Datenblöcke übertragen werden. Hierbei kann es sich beispielsweise um eine einmalige Freigabe handeln, so daß dem Expertensystem signalisiert wird, daß ein bestimmter Datenblock stets automatisch abrufbar ist, alternativ hierzu kann das Expertensystem auch eine direkte Anfrage bezüglich der Freigabe eines bestimmten Datenblockes stellen. Der Speicherbereich kann aber auch in der dritten Datenverarbeitungseinrichtung, also z.B. den zentralen Server vorgesehen sein.

Bei solchen Daten kann es sich beispielsweise um ein medizinisches Datenbuch handeln, in welches der Patient kontinuierlich entsprechende Informationen einträgt, um eine Medikamentenliste, in welcher aufgeführt ist, welche Medikamente der Patient in welcher Menge zu sich nimmt, und die über mehrere Tage aufgenommenen Puls- und Blutdruckwerte etc. Alternativ oder zusätzlich kann erfindungsgemäß der oder ggfs. ein weiterer Speicherbereich auch arztseitig vorgesehen sein, wobei die patientenspezifischen Daten automatisch in Abhängigkeit einer vorzugsweise während der Aufnahme der Anamnesedaten aufgenommenen Patientenkennung oder dergleichen abfragbar und im Rahmen der Ausgabe der Anamnesedaten mit ausgebbar sind. Bei diesen Daten kann es sich beispielsweise um die elektronische Patientenkartei handeln, in welcher sämtliche relevanten, arztseitig vorliegenden Patientendaten, z.B. Krankengeschichte, Versicherungsnummer etc. enthalten sind. Diese wird beispielsweise bei der Ausgabe der Anamnesedaten, wenn der Arzt diese also abruft und sich am Monitor anzeigen läßt, mit ausgegeben. Als Patientenkennung kann beispielsweise die e-mail oder Internetadresse des Patienten verwendet werden, daneben kann natürlich auch im Rahmen der Kommunikation Patient-Expertensystem automatisch eine entsprechende Patientenkennung mit übertragen werden, hier sind keine Grenzen gesetzt.

Des weiteren muß für den Fall, daß das Expertensystem im Rahmen der Aufnahme der Anamnesedaten eine Notlage erkennt, die ein sofortiges Handeln erfordert, sichergestellt sein, daß die aufgenommene Anamnesedaten bzw. die Patientenanfrage nicht unbemerkt bis zur zu einem unbestimmten Zeitpunkt erfolgenden Abfrage durch den Arzt bleibt. Hierzu kann erfindungsgemäß das Expertensystem in Abhängigkeit der patientenseitig gegebenen Daten zur unmittelbaren Weiterleitung der aufgenommenen Daten an wenigstens eine mit der ersten Datenverarbeitungseinrichtung kommunizierende weitere Datenverarbeitungseinrichtung, eine Ausgabeeinrichtung und/oder eine externe Notrufzentrale ausgebildet sein. Gemäß dieser erfindungsgemäßen Weiterbildung leitet das Expertensystem bzw. die erste Datenverarbeitungseinrichtung dann, wenn eine solche Notlage erkannt wird, was durch das Expertensystem ohne weiteres möglich ist, die Daten bzw. Anfragen sofort an den Arzt, der beispielsweise in einem anderen Behandlungszimmer ist, weiter, alternativ kann die Notlage auch einer Sprechstundenhilfe oder dergleichen signalisiert werden. Daneben ist auch eine sofortige Weiterleitung an eine externe Notrufzentrale möglich, was wiederum über eine Kommunikationsleitung erfolgt, so daß sofort von extern Hilfe kommen kann. Letzteres ist insbesondere dann relevant, wenn die Patientenanfrage außerhalb der Sprechstundenzeiten des Arztes eingeht.

Für den Fall, daß der Arzt beispielsweise urlaubsbedingt abwesend ist, kann das Expertensystem bzw. die erste Datenverarbeitungseinrichtung im Bedarfsfall zur Weiterleitung der Patientenanfrage an eine externe, bei einem anderen Arzt vorgesehene erste Datenverarbeitungseinrichtung und/oder zur Ausgabe und Übermittlung einer bedarfsfallabhängigen Mitteilung, beispielsweise betreffend die Abwesenheit, an den Patienten ausgebildet sein. Die Anfrage wird also entweder an einen vertretenden Kollegen, der ebenfalls ein entsprechendes System installiert hat, weitergeleitet, gleichzeitig oder alternativ dazu wird dem Patienten die Abwesenheit des Arztes wie auch ggfs. sein Vertreter mitgeteilt. Das Expertensystem selbst kann ein neuronales Netz oder ein Bayes'sches Netz umfassen, daneben sind natürlich auch andere Systemkonfigurationen denkbar. Aus Sicherheitsgründen können die Daten, vornehmlich die Patienteninformationen und die patientenspezifischen Daten, auch verschlüsselt übertragen werden.

Neben dem System selbst betrifft die Erfindung ferner ein Verfahren zum Aufnehmen relevanter, insbesonderer medizinisch relevanter Anamnesedaten eines Patienten, bei dem der Patient mittels einer patientenseitig vorgesehenen Datenverarbeitungseinrichtung mit zugeordnetem Ausgabemedium mit einer arztseitig vorgesehenen Datenverarbeitungseinrichtung mit zugeordnetem Ausgabemedium über eine Kommunikationsleitung - zweckmäßigerweise unter Verwendung eines Passwortes zum Aufbau einer Passwort-geschützten Verbindung - kommuniziert, wobei ein der arztseitigen Datenverarbeitungseinrichtung zugeordnetes Expertensystem gezielt relevante Anamnesedaten interaktiv abfragt, wobei die Daten gesammelt und im Bedarfsfall dem Arzt ausgegeben werden.

Weitere zweckmäßige Ausgestaltungen des erfindungsgemäßen Verfahrens sind den abhängigen Unteransprüchen zu entnehmen.

Weitere Vorteile, Merkmale und Einzelheiten der Erfindung ergeben sich aus dem im folgenden beschriebenen Ausführungsbeispiel sowie anhand der Zeichnungen. Dabei zeigen:
- Fig. 1: eine Systemkonfiguration einer ersten Ausführung,
und
- Fig. 2: eine Systemkonfiguration einer zweiten Ausführung.

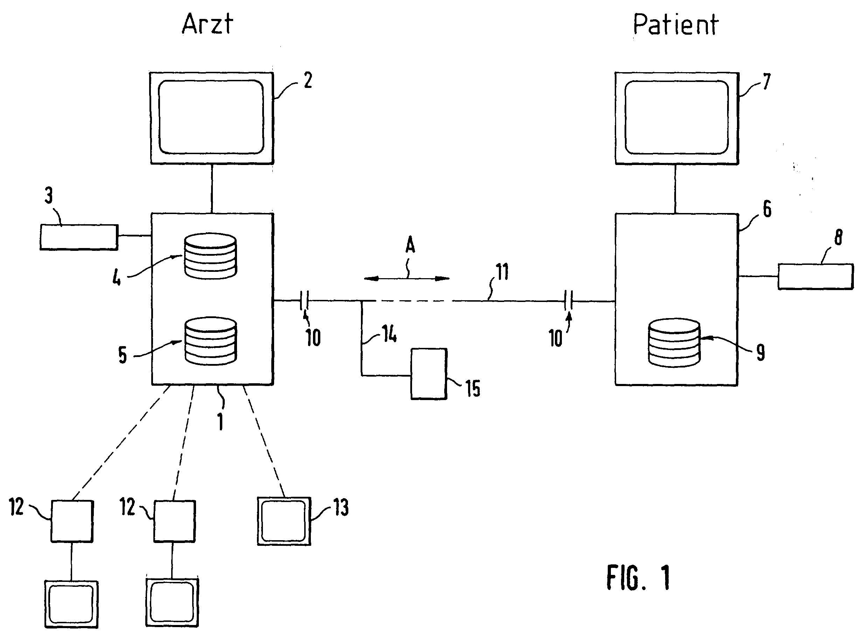

In Figur 1 ist in Form einer Prinzipskizze das erfindungsgemäßge System gezeigt. Dieses besteht aus einer bei dem Arzt stationierten ersten Datenverarbeitungseinrichtung 1 mit zugeordnetem Ausgabemedium 2 in Form eines Monitors und einem Dateneingabemedium in Form einer Tastatur 3. In der Datenverarbeitungseinrichtung 1 ist ein Expertensystem 4 implementiert. Bei diesem handelt es sich um ein wissensbasiertes, intelligentes System, in dem medizinisches Fachwissen abgelegt ist. Dieses System ist zum Verknüpfen von ihm gegebener sowie vorhandener Information und zur Erzeugung von Schlußfolgerungen aus den vorliegenden Informationen ausgebildet. Dieses Expertensystem kann beispielsweise ein neuronales Netz oder ein Bayes'sches Netz umfassen.

Ferner ist ein Speicherbereich 5 vorgesehen, in dem patientenspezifische Daten wie beispielsweise die Patientenkarteien in elektronischer Form abgelegt sind. Auf diesen Speicherbereich 5, also diese Datenbank, hat das ebenfalls nach Art einer Datenbank strukturierte Expertensystem 4 im Bedarfsfall Zugriff.

Das System umfaßt ferner eine zweite Datenverarbeitungseinrichtung 6, die bei dem Patienten installiert ist. Auch dieser ist ein Ausgabemedium 7 in Form eines Monitors und ein Eingabemedium in Form einer Tastatur 8 zugeordnet. Die zweite Datenverarbeitungseinrichtung 6 weist ebenfalls einen Speicherbereich 9 auf, in dem patientenspezifische Daten in Form einer Datenbank abgelegt sind. Diese Datenbank ist vom Patienten selbst zu pflegen und kann beispielsweise Medikamentenlisten, ein medizinisches Tagebuch, Meßdaten des Pulses, Blutdruck, EKG etc. enthalten.

Die beiden Datenverarbeitungseinrichtungen 1, 6 können über entsprechende Schnittstellen 10 über eine Kommunikationsleitung 11 (leitungsgebunden oder drahtlos) miteinander kommunizieren, wobei hier ein bidirektionaler Datenaustausch möglich ist, um eine interaktive Frage-Antwort-Kommunikation zu ermöglichen, wie durch den Doppelpfeil A angedeutet ist. Die Arbeitsweise ist nun derart, daß sich der Patient mittels seiner Datenverarbeitungseinrichtung 6 über die Kommunikationsleitung 11 bei der Datenverarbeitungseinrichtung 1 des Arztes einwählt, wozu er beispielsweise zunächst ein Passwort einzugeben hat, um die Verbindung aufzubauen. Die Kommunikation kann dabei über das Internet erfolgen, alternativ können die Daten auch als e-mail verschickt werden. Nach dem Einwählen beginnt der interaktive Kommunikationsprozess zwischen den beiden Datenverarbeitungseinrichtungen, wobei dieser über das Expertensystem gesteuert erfolgt. Das Expertensystem 4 stellt abhängig vom patientengeschilderten medizinischen Problem gezielt Fragen, die dem Patienten am Ausgabemedium 7 angezeigt werden und die dieser mittels der Tastatur 8 beantwortet. Diese Fragen, die das Expertensystem aufgrund des abgelegten Fachwissens formulieren kann, dienen zur gezielten Ermittlung relevanter Anamnesedaten, die der Patient einzugeben hat. Das Expertensystem 4 ist in der Lage, die vom Patienten gegebene Information zu analysieren, um unvollständige Informationen oder mehrdeutige Informationen zu erkennen und ggfs. nachzufragen. Ferner erfragt das Expertensystem 4 sämtliche relevanten Anamnesedaten, die der Arzt zur Beurteilung benötigt.

Im Rahmen der interaktiven Kommunikation kann der Patient auch von sich aus entsprechende Daten aus seinem Speicherbereich 9 zur Übertragung an das Expertensystem 4 freigeben, alternativ kann das Expertensystem auch automatisch auf bestimmte Datensätze, die möglicherweise bereits vorher einmal freigegeben wurden, zugreifen. Auch diese Daten werden dem Arzt im Rahmen der Abarbeitung der Patientenanfrage mit ausgegeben. Hat das Expertensystem sämtliche geforderten Informationen erhalten, wird die Verbindung beendet. Das Expertensystem 4 ist des weiteren in der Lage, die gegebene Patienteninformation, sofern diese teilweise inkorrekt oder mehrdeutig ist, in medizinisch eindeutige Aussagen "zu übersetzen", d.h., es ist imstande, den Informationsgehalt dieser Daten zu erkennen, auszuwerten und entsprechende korrekte, aussagekräftige Informationen für den Arzt zu formulieren.

Der Arzt hat nun die Möglichkeit, die gesammelten Daten bei Bedarf, beispielsweise nach Praxisschluß, abzurufen, was mittels der Tastatur 3 erfolgt. Die Daten werden dem Arzt am Ausgabemedium 2 angezeigt, wobei hierbei nicht nur die vom Patienten gelieferten, ggfs. "übersetzten" Daten ausgegeben werden, sondern auch die im Speicherbereich 5 abgelegten patientenspezifischen Daten, auf welche das Expertensystem zugreifen kann, beispielsweise über eine Patientenkennung, die dem Expertensystem 4 im Rahmen der Patientenkommunikation mitgeteilt wurde. Anhand der ausgegebenen Information kann der Arzt nun seinen medizinischen Rat formulieren und diesen beispielsweise wiederum über die Kommunikationsleitung an den Patienten schicken. Alternativ dazu ist selbstverständlich auch ein persönlicher Kontakt am darauffolgenden Tag möglich, sofern dies erforderlich ist.

Das Expertensystem 4 ist ferner zu jedem Zeitpunkt in der Lage, bei Erkennen einer Notlage, die ein sofortiges Handeln ermöglicht, die Patientenanfrage unmittelbar an den Arzt weiterzuleiten, beispielsweise an das Ausgabemedium 2 oder an entsprechende andere Datenverarbeitungseinrichtungen 12 oder andere Ausgabemedien 13. Auf diese Weise ist es möglich, den Arzt oder eine Sprechstundenhilfe sofort von einer gegebenen Notlage zu informieren, die ein Abarbeiten der Patientenanfrage zu einem viel späteren Zeitpunkt ausschließt. Alternativ besteht auch die Möglichkeit, daß die Patientenanfrage über die Kommunikationsleitung 14 an eine Notrufzentrale 15, beispielsweise ein Krankenhaus oder dergleichen, weitergeleitet wird, insbesondere dann, wenn die Patientenanfrage nach Praxisschluß eingeht.

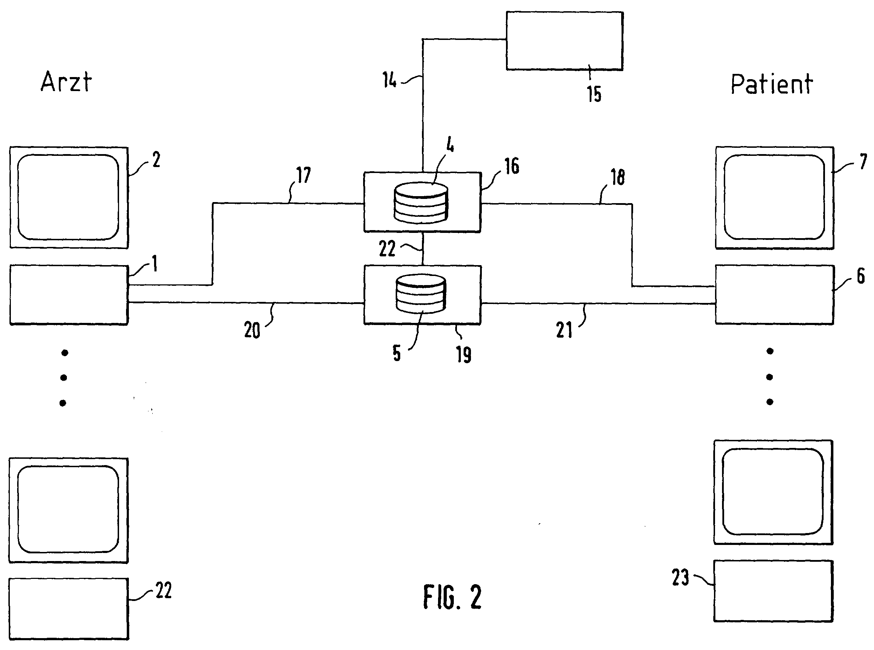

Fig. 2 zeigt eine weitere Konfigurationsmöglichkeit des erfindungsgemäßen Systems. Bei diesem ist das Expertensystem 4 nicht wie bei dem System gemäß Fig. 1 in der arztseitigen Datenverarbeitungseinrichtung vorgesehen, sondern in einer externen, dritten Datenverarbeitungseinrichtung 16, die mit den ersten und zweiten Datenverarbeitungseinrichtungen 1, 6 über entsprechende Kommunikationsleitungen 17, 18 verbunden ist. Diese dritte Datenverarbeitungseinrichtung 16 steht zu den anderen beiden extern, es kann sich beispielsweise um einen zentralen Server oder dergleichen handeln. Die Kommunikation erfolgt in diesem Fall über diese dritte Datenverarbeitungseinrichtung 16. Der Patient kann sich dabei unmittelbar an der dritten Datenverarbeitungseinrichtung 16 einwählen, die dann auf Anfrage die Verbindung zur jeweiligen Datenverarbeitungseinrichtung 1 bei dem gewünschten Arzt herstellt, alternativ kann der Betrieb auch derart sein, daß sich der Patient bei der arztseitigen Datenverarbeitungseinrichtung 1 einwählt, die dann die entsprechende Kommunikationsverbindung zur dritten Datenverarbeitungseinrichtung 16 herstellt. Hier sind sämtliche Kommunikationswege denkbar.

Wie der Figur ferner zu entnehmen ist, ist im gezeigten Beispiel der Speicherbereich 5 mit patientenspezifischen Daten in einer vierten Datenverarbeitungseinrichtung 19 vorgesehen, die mit der ersten und der zweiten Datenverarbeitungseinrichtung 1, 6 über entsprechende Kommunikationsleitungen 20, 21 in Verbindung steht. Ferner ist eine Kommunikationsleitung 22 zur dritten Datenverarbeitungseinrichtung 16 vorgesehen. Es ist also eine vollständige Vernetzung gegeben. Alternativ dazu ist es natürlich auch möglich, den Speicherbereich 5 in der dritten Datenverarbeitungseinrichtung 16 vorzusehen, so daß in dieser sowohl das Expertensystem 4 als auch dieser Speicherbereich 5 integriert sind. In diesem Fall erfolgt die Kommunikation dann ausschließlich über die Kommunikationsleitungen 17, 18, wobei das Expertensystem 4 und der Speicherbereich 5 natürlich über interne Kommunikationsleitungen miteinander in Verbindung stehen.

Bei der hier gezeigten Systemkonfiguration ist die Notrufzentrale 15 über die Kommunikationsleitung 14 mit der dritten Datenverarbeitungseinrichtung 16 verbunden, da von dieser aus bei Erkennen einer Notlage durch das Expertensystem 4 die Notrufzentrale angesprochen wird.

Wie der Figur ferner zu entnehmen ist, können in das System beliebig viele Arztteilnehmer und Patiententeilnehmer eingebunden werden, wie durch die weiteren Datenverarbeitungseinrichtungen samt Ausgabemedien 22, 23 angedeutet ist. Diese sind über entsprechende Kommunikationsleitungen ebenfalls mit der zentralen dritten, ggf. auch der vierten Datenverarbeitungseinrichtung verbunden, so daß jeder eingebundene Patiententeilnehmer über das Expertensystem mit jedem eingebundenen Arzt und umgekehrt kommunizieren kann.

## Patentansprüche

1. System zur Kommunikation zwischen einem Arzt und einem Patienten über eine Kommunikationsleitung, umfassend eine erste arztseitig vorgesehene Datenverarbeitungseinrichtung (1) mit zugeordnetem Ausgabemedium (2) und eine zweite patientenseitig vorgesehene Datenverarbeitungseinrichtung (6) mit zugeordnetem Ausgabemedium (7), die kanalgebunden miteinander kommunizieren und denen ein Expertensystem (4) zugeordnet ist, das zur interaktiven kanalgebundenen Abfrage und Aufnahme relevanter, insbesondere medizinisch relevanter und vom Patienten entsprechend der vom Expertensystem (4) formulierten, an die zweite Datenverarbeitungseinrichtung (6) gegebenen Anfragen einzugebener Anamnesedaten des Patienten ausgebildet ist, welche bei Bedarf dem Arzt über dessen Ausgabemedium ausgebbar sind.

2. System nach Anspruch 1, **dadurch gekennzeichnet**, daß das Expertensystem (4) in der ersten, der zweiten oder einer dritten externen und mit der ersten und der zweiten Datenverarbeitungseinrichtung (1, 6) kanalgebunden kommunizierenden Datenverarbeitungseinrichtung (16) implementiert ist.

3. System nach Anspruch 1 oder 2, **dadurch gekennzeichnet**, daß das Expertensystem (4) zur Formulierung einer jeweiligen Anfrage in Abhängigkeit einer oder mehrerer vorher patientenseitig gegebener Daten und/oder Anfragen ausgebildet ist.

4. System nach einem der vorangehenden Ansprüche, **dadurch gekennzeichnet**, daß das Expertensystem (4) zum Erkennen und/oder Formulieren und Ausgeben medizinisch eindeutiger Anamnesedaten auf Basis der vom Patienten eingegebenen Daten ausgebildet ist.

5. System nach einem der vorangehenden Ansprüche, **dadurch gekennzeichnet**, daß wenigstens einer der Datenverarbeitungseinrichtungen (1, 6) ein Speicherbereich (5, 9) enthaltend patientenspezifische Daten zugeordnet ist, oder daß eine den Speicherbereich (5, 9) aufweisende und zur ersten und zweiten Datenverarbeitungseinrichtung externe vierte Datenverarbeitungseinrichtung (19) vorgesehen ist.

6. System nach Anspruch 5, **dadurch gekennzeichnet**, daß der Speicherbereich (9) patientenseitig vorgesehen ist, wobei die Daten zumindets teilweise automatisch von der ersten Datenverarbeitungseinrichtung (1) bzw. dem Expertensystem (4) abfragbar sind, und/oder zumindest teilweise bei Bedarf vom Patienten zur Übertragung freigebbar sind.

7. System nach Anspruch 5 oder 6, **dadurch gekennzeichnet**, daß der oder gegebenenfalls ein weiterer Speicherbereich (5) arztseitig vorgesehen ist, wobei die patientenspezifischen Daten automatisch in Abhängigkeit einer vorzugsweise während der Aufnahme der Anamnesedaten aufgenommener Patientenkennung o.dgl. abfragbar und im Rahmen der Ausgabe der Anamnesedaten mit ausgebbar sind.

8. System nach Anspruch 5 oder 6, **dadurch gekennzeichnet**, daß der Speicherbereich (5, 9) in der dritten Datenverarbeitungseinrichtung (16) vorgesehen ist.

9. System nach einem der vorangehenden Ansprüche, **dadurch gekennzeichnet**, daß die aufgenommenen Daten in Abhängigkeit der patientenseitig gegebenen Daten unmittelbar an wenigstens eine mit der ersten Datenverarbeitungseinrichtung (1) kommunizierende weitere Datenverarbeitungseinrichtung (12), eine Ausgabeeinrichtung (13) und/oder eine externe Notrufzentrale (15) weiterleitbar sind.

10. System nach einem der vorangehenden Ansprüche, **dadurch gekennzeichnet,** daß die Patientenanfrage im Bedarfsfall an eine externe, bei einem anderen Arzt vorgesehene erste Datenverarbeitungseinrichtung (12) weiterleitbar ist, und/oder daß eine bedarfsfallabhängige Mitteilung an den Patienten ausgebbar ist.

11. System nach einem der vorangehenden Ansprüche, **dadurch gekennzeichnet**, daß das Expertensystem (4) ein neuronales Netz oder ein Bayes'sches Netz umfaßt.

12. System nach einem der vorangehenden Ansprüche, **dadurch gekennzeichnet**, daß Mittel zum Verschlüsseln und Entschlüsseln der zu übertragenden bzw. empfangenen Daten vorgesehen sind.

13. Verfahren zum Aufnehmen relevanter, insbesondere medizinisch relevanter Anamnesedaten eines Patienten, bei dem der Patient mittels einer patientenseitig vorgesehenen Datenverarbeitungseinrichtung mit zugeordnetem Ausgabemedium mit einer arztseitig vorgesehenen Datenverarbeitungseinrichtung mit zugeordnetem Ausgabemedium über eine Kommunikationsleitung kommuniziert, wobei ein innerhalb des Kommunikationsbetriebes aktives Expertensystem gezielt relevante Anamnesedaten interaktiv abfragt, wobei die Daten gesammelt und im Bedarfsfall dem Arzt über dessen Ausgabemedium ausgegeben werden.

14. Verfahren nach Anspruch 12, **dadurch gekennzeichnet**, daß ein in der ersten oder der zweiten Datenverarbeitungseinrichtung implementiertes Expertensystem verwendet wird, oder daß ein in einer dritten, externen Datenverarbeitungseinrichtung implementiertes Expertensystem verwendet wird, welche kanalgebunden mit der ersten und der zweiten Datenverarbeitungseinrichtung kommuniziert.

15. Verfahren nach Anspruch 13 oder 14, **dadurch gekennzeichnet**, daß das Expertensystem eine jeweilige Anfrage in Abhängigkeit einer oder mehrerer vorher patientenseitig gegebener Daten und/oder Anfragen formuliert.

16. Verfahren nach einem der Ansprüche 13 bis 15, **dadurch gekennzeichnet**, daß das Expertensystem auf Basis der vom Patienten eingegebenen Daten medizinisch eindeutige Anamnesedaten erkennt und/oder formuliert und ausgibt.

17. Verfahren nach einem der Ansprüche 13 bis 16, **dadurch gekennzeichnet**, daß patientenspezifische Daten, die in wenigstens einem einer der Datenverarbeitungseinrichtungen zugeordneten Speicherbereich oder in einem in einer vierten, externen Datenverarbeitungseinrichtung vorgesehenen Speicherbereich abgelegt sind, im Rahmen der Aufnahme der Anamnesedaten und/oder der Ausgabe der Anamnesedaten an den Arzt übertragen und dem Arzt ausgegeben werden werden.

18. Verfahren nach Anspruch 17, **dadurch gekennzeichnet**, daß der Speicherbereich patientenseitig vorgesehen ist, wobei die Daten zumindets teilweise automatisch von der ersten Datenverarbeitungseinrichtung bzw. dem Expertensystem abgefragt werden, und/oder zumindest teilweise bei Bedarf vom Patienten zur Übertragung freizugeben sind.

19. Verfahren nach Anspruch 17 oder 18, **dadurch gekennzeichnet**, daß der oder gegebenenfalls ein weiterer Speicherbereich arztseitig vorgesehen ist, wobei die patientenspezifischen Daten automatisch in Abhängigkeit einer vorzugsweise während der Aufnahme der Anamnesedaten aufgenommener Patientenkennung o.dgl. abgefragt und im Rahmen der Ausgabe der Anamnesedaten mit ausgegeben werden.

20. Verfahren nach einem der Ansprüche 17 bis 19, **dadurch gekennzeichnet**, daß eine dritte Datenverarbeitungseinrichtung mit integriertem Speicherbereich vorgesehen ist.

21. Verfahren nach einem der Ansprüche 13 bis 20, **dadurch gekennzeichnet**, daß das Expertensystem in Abhängigkeit der patientenseitig gegebenen Daten die aufgenommenen Daten unmittelbar an wenigstens eine mit der ersten Datenverarbeitungseinrichtung kommunizierende weitere Datenverarbeitungseinrichtung, eine Ausgabeeinrichtung und/oder eine externe Notrufzentrale weiterleitet.

22. Verfahren nach einem der Ansprüche 13 bis 21, **dadurch gekennzeichnet**, daß das Expertensystem bzw. die erste Datenverarbeitungseinrichtung im Bedarfsfall die Patientenanfrage an eine externe, bei einem anderen Arzt vorgesehene erste Datenverarbeitungseinrichtung weiterleitet, und/oder eine bedarfsfallabhängige Mitteilung an den Patienten ausgibt.

23. Verfahren nach einem der Ansprüche 13 bis 22, **dadurch gekennzeichnet**, daß die Daten verschlüsselt übertragen werden.
